(19) Europäisches Patentamt
European Patent Office
Office européen des brevets

(11) Veröffentlichungsnummer: **0 199 262 B1**

(12) # EUROPÄISCHE PATENTSCHRIFT

(45) Veröffentlichungstag der Patentschrift: **27.02.91**

(51) Int. Cl.5: **C07D 249/04**, A61K 31/41

(21) Anmeldenummer: **86105188.6**

(22) Anmeldetag: **15.04.86**

Die Akte enthält technische Angaben, die nach dem Eingang der Anmeldung eingereicht wurden und die nicht in dieser Patentschrift enthalten sind.

(54) **Fluorierte Benzyltriazolverbindungen.**

(30) Priorität: **18.04.85 CH 1663/85**

(43) Veröffentlichungstag der Anmeldung:
**29.10.86 Patentblatt 86/44**

(45) Bekanntmachung des Hinweises auf die Patenterteilung:
**27.02.91 Patentblatt 91/09**

(84) Benannte Vertragsstaaten:
**AT BE CH DE FR GB IT LI LU NL SE**

(56) Entgegenhaltungen:
**EP-A- 0 114 347**
**GB-A- 1 511 195**

(73) Patentinhaber: **CIBA-GEIGY AG**
**Klybeckstrasse 141**
**CH-4002 Basel(CH)**

(72) Erfinder: **Meier, René**
**Rickenbacherstrasse 76**
**CH-4463 Buus(CH)**

(74) Vertreter: **Zumstein, Fritz, Dr. et al**
**Bräuhausstrasse 4**
**D-8000 München 2(DE)**

**EP 0 199 262 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue fluorierte 1-(α-Phenylalkyl)-1H-1,2,3-triazolverbindungen der Formel

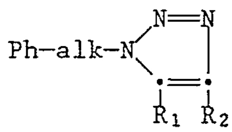 (I),

worin Ph in o-Stellung durch Fluor und gegebenenfalls zusätzlich durch H bis und mit 2 Chloratome, 1 Fluor - und 1 Chloratom oder bis und mit 2 Fluoratome substituiertes Phenyl bedeutet, alk für Methylen steht, $R_1$ Wasserstoff ,Carbamyl, N-$C_2$-$C_5$-Alkanoylcarbamyl oder N,N-Di-$C_1$-$C_4$-alkylcarbamyl ist und $R_2$ Carbamyl, N-$C_2$-$C_5$-Alkanoylcarbamyl oder N,N-Di-$C_1$-$C_4$-alkylcarbamyl darstellt, Verfahren zur Herstellung dieser Verbindungen,diese Verbindungen enthaltende pharmazeutische Präparate und die Verwendung dieser Verbindungen.

Insgesamt können bis und mit 3 Halogensubstituenten vorhanden sein, zusätzlich zum o-Fluorsubstituenten 1 oder 2 Fluoratom(e).Ein einzelnes zusätzliches Halogenatom ist dabei insbesondere in 6-Stellung gebunden.

N-$C_2$-$C_5$-Alkanoylcarbamyl ist z.B. N-Acetyl- oder N-Pivaloylcarbamyl; N,N-Di-$C_1$-$C_4$-alkylcarbamyl ist z.B. N,N-Dimethylcarbamyl oder N,N-Diäthylcarbamyl.

Die Verbindungen der Formal I besitzen wertvolle pharmakologische Eigenschaften, insbesondere eine ausgebrägte antikonvulsive Wirksamkeit,die sich beispielsweise an der Maus anhand eines deutlichen Metrazol-Antagonismus im Dosisbereich von etwa 30 bis etwa 300 mg/kg p.o. sowie an Maus und Ratte anhand einer ausgeprägten Schutzwirkung gegen durch Elektroschock ausgelöste Konvulsionen im Dosisbereich von etwa 1 bis etwa 50, in den meisten Fällen von etwa 1 bis etwa 25 mg/kg p.o. zeigen lässt. In diesem Modell wurden beispielsweise die folgenden Werte für die effektive Dosis $ED_{50}$ in mg/kg p.o. erhalten (1 h Vorapplikation):

1-(2,6-Difluorbenzyl)-1H-1,2,3-triazol-4-carboxamid: 17 (Maus) bzw. 8 (Ratte);
1-(o-Fluorbenzyl)-1H-1,2,3-triazol-4-carboxamid: 17 (Maus, Ratte);
1-(2,6-Difluorbenzyl)-1H-1,2,3-triazol-4,5-dicarboxamid: 4 (Maus, Ratte);
1-(6-Chlor-2-fluor-benzyl)-1H-1,2,3-triazol-4,5-dicarboamid: 7 (Maus) bzw. 10 (Ratte);
1-(o-Fluorbenzyl)-1H-1,2,3-triazol-4,5-dicarboxamid: 6 (Maus) bzw. 10 (Ratte);
1-(6-Chlor-2-fluor-benzyl)-1H-1,2,3-triazol-4-carboamid: 11 (Maus);
1-(2,5-Difluorbenzyl)-1H-1,2,3-triazol-4,5-dicarboamid: 6 (Maus).

In der europäischen Patentanmeldung Nr. 114 347 sind im Phenylteil durch u.a. Halogen substituierte 1-Phenylalkyl-1H-1,2,3-triazol-4-carboxamide und -4,5-dicarboamide mit antikonvulsiven Eigenschaften offenbart. Die dort spezifisch genannten 5-unsubstituierten Verbindungen weisen als Halogensubstituenten durchwegs Chlor auf. Ist der Halogensubstituent hingegen Fluor, dann befindet sich in 5-Stellung des Triazolringes eine Aminogruppe. Die erfindungsgemäss bereitgestellten, im Phenylteil stets durch Fluor substituierten 1-Benzyl-1H-1,2,3-triazol-4-carboxamide und -4,5-dicarboxamide weisen gegenüber den vorbekannten Verbindungen den Vorteil einer grösseren Wirksamkeit bzw. einer längeren Wirkungsdauer auf. So ergaben sich in obigem Modell z.B. folgende $ED_{50}$-Werte: 1-(o-Chlorbenzyl)-1H-1,2,3-triazol-4-carboxamid: 26 (Maus) bzw. 25 (Ratte) und 1-(o-Chlorbenzyl)-1H-1,2,3-triazol-4,5-dicarboxamid: 40 (Maus) bzw. 43 (Ratte).

Die erfindungsgemäss bereitgestellten Verbindungen sind dementsprechend vorzüglich geeignet zur Behandlung von Konvulsionen verschiedenartiger Genese, beispielsweise zur Behandlung der Epilepsie, und können als antikonvulsive, beispielsweise antiepileptische, Arzneiwirkstoffe verwendet werden.

Die Erfindung betrifft in erster Linie Verbindungen der Formel (I), worin Ph in o-Stellung durch Fluor und gegebenenfalls zusätzlich durch 1 Chloratom, 1 Fluor- und 1 Chloratom oder bis und mit 2 Fluoratome substi-tuiertes Phenyl, wie o-Fluorphenyl, 2,3-, 2,4-, 2,5- oder 2,6-Difluorphenyl, 2-Chlor-6-fluorphenyl, ferner 2,4,6-Trifluorphenyl, bedeutet, alk für Methylen steht, $R_1$ Wasserstoff oder einen Rest $R_2$ bedeutet und $R_2$ Carbamyl oder in zweiter Linie N,N-Di-C1-C4-alkylcarbamyl,wie N,N-Dimethylcarbamyl ,bedeutet, beispielsweise Verbindungen der Formel (I), worin Ph o-Fluorphenyl, 2,3-, 2,4-, 2,5- oder 2,6-Difluorphenyl oder 6-Chlor-2-fluorphenyl bedeutet, alk für Methylen steht, $R_1$ Wasserstoff oder unsubsti-tuiertes Carbamyl bedeutet und $R_2$ unsubstituiertes Carbamyl darstellt.

Die Erfindung betrifft insbesondere Verbindungen der Formel (I), worin Ph o-Fluorphenyl oder 2,6-Difluorphenyl bedeutet, alk Methylen darstellt, $R_1$ Wasserstoff oder unsubstituiertes Carbamyl bedeutet und

$R_2$ unsubstituiertes Carbamyl ist.

Die Erfindung betrifft vorzugsweise Verbindungen der Formel (I), worin Ph 2,6-Difluorphenyl bedeutet, alk Methylen darstellt, $R_1$ Wasserstoff oder einen Rest $R_2$ bedeutet und $R_2$ Carbamyl oder in zweiter Linie N-$C_2$-$C_5$-Alkanoylcarbamyl, wie N- Acetycarbamyl, oder N,N-Di-$C_1$-$C_4$-alkylcarbamyl, wie N,N- Dimethylcarbamyl, darstellt.

Die Erfindung betrifft in allererster Linie Verbindungen der Formel (I), worin Ph o-Fluorphenyl, 2,5-Difluorphenyl, 2,6-Difluorphenyl oder 2-Chlor-6-fluor-phenyl bedeutet, alk für Methylen steht und $R_1$ und $R_2$beide Carbamyl darstellen, ferner Verbindungen der Formel I, worin Ph 2,6-Difluorphenyl, alk Methylen, $R_1$ Wasserstoff oder Garbamyl und $R_2$ Carbamyl bedeutet.

Die Verbindungen der Formel können nach an sich bekannten Methoden hergestellt werden, beispielsweise indem man

a) eine Verbindung der Formel

$$Ph - alk - N_3 \qquad (II)$$

mit einer Verbindung der Formel

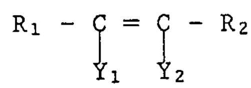

(III),

worin $Y_1$ Hydroxy und $Y_2$ Wasserstoff darstellt oder $Y_1$ und $Y_2$ gemeinsam eine zusätzliche Bindung bedeuten, oder einem Salz und/oder Tautomeren davon umsetzt oder

b) eine Verbindung der Formel

$$Ph - alk - Z \qquad (IV),$$

worin Z reaktionsfähiges verestertes Hydroxy bedeutet, mit einem 1H-1,2,3-Triazolderivat der Formel

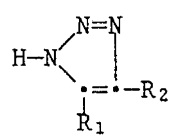

(V)

oder einem Salz davon umsetzt oder

c) Zur Herstellung von Verbindungen der Formel I, worin entweder $R_1$ Wasserstoff, Carbamyl, N-$C_2$-$C_5$-Alkanoylcarbamyl oder N,N-Di-$C_1$-$C_4$-alkylcarbamyl ist und $R_2$ Carbamyl oder N,N-Di-$C_1$-$C_4$-alkylcarbamyl ist oder $R_1$ Carbamyl oder N,N-Di-$C_1$-$C_4$ alkylcarbamyl ist und $R_2$ Carbamyl, N-$C_2$-$C_5$-Alkanoylcarbamyl oder N,N-Di-$C_1$-$C_4$-alkylcarbamyl ist, in einer Verbindung der Formel

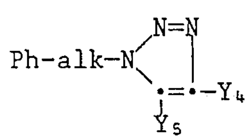

(VI),

worin $Y_4$ einen in Carbamyl oder N,N-Di-$C_1$-$C_4$-alkylcarbamyl überführbaren Rest $Y_A$ und $Y_5$ eine Gruppe $R_1$ oder einen in Carbamyl oder N,N-Di-$C_1$-$C_4$-alkylcarbamyl überführbaren Rest $Y_B$ oder $Y_4$ eine Gruppe $R_2$ und $Y_5$ einen in Carbamyl oder N,N-Di-$C_1$-$C_4$-alkylcarbamyl überführbaren Rest Y darstellt, $Y_A$ und/oder $Y_B$ in Carbamyl oder N,N-Di-$C_1$-$C_4$-alkylcarbamyl überführt, erforderlichenfalls ein erhaltenes Isomerengemisch in die Komponenten auftrennt und das Isomere der Formel (I) isoliert und gewünschtenfalls eine verfahrensgemäss erhältliche Verbindung der Formel I eine andere Verbindung der Formel (I) umwandelt und/oder ein verfahrensgemäss erhältliches Enantiomeren- bzw. Diastereomerengemisch in die Komponenten aufspaltet.

Als Ausgangsstoffe der Formel (III) für die Verfahrensvariante a) und Tautomere derselben kommen beispielsweise Verbindungen der Formeln $R_1-C\equiv C-R_2$ (IIIa) und $R_1-C(=O)-CH_2-R_2$ (IIIb) in Betracht. Salze derselben sind beispielsweise Alkalimetall-, z.B. Natriumsalze von Verbindungen der Formel (IIIa), die aus

diesen und Alkalimetallalkanolaten, z.B. Natriummethanolat, erhalten werden können.

Die Umsetzung von Verbindungen II und III erfolgt in üblicher Weise, vorteilhaft in einem inerten Lösungsmittel, erforderlichenfalls in Gegenwart eines Kondensationsmittels und/oder bei erhöhter Temperatur. Inerte Lösungsmittel sind beispielsweise aromatische oder araliphatische Kohlenwasserstoffe, wie Benzol oder Toluol, oder Aether, wie Tertiärbutoxymethan, Tetrahydrofuran oder Dioxan.

Bevorzugte Ausführungsformen dieses Verfahrens sind beispielsweise die Umsetzung eines Azides der Formel (II) mit einer Verbindung der Formel (IIIa) in Benzol oder Dioxan bei etwa 60-120 °C, vorzugsweise Siedetemperatur.

Die Ausgangsstoffe der Formel (III) und teilweise der Formel (II) sind bekannt. Neue Ausgangsstoffe der Formel (II) können in Analogie zur Bildungsweise der bekannten erhalten werden, beispielsweise indem man eine Verbindung der Formel Ph-alk-Z (IV), worin Z reaktionsfähiges verestertes Hydroxy, wie Halogen, z.B. Chlor, Brom oder Iod, oder Sulfonyloxy, wie $C_1$-$C_4$-Alkansulfonyloxy oder gegebenenfalls substituiertes Benzolsulfonyloxy, wie Methan-, Aethan-, Benzol-, p-Toluol- oder p-Brombenzolsulfonyloxy, oder Fluorsulfonyloxy bedeutet, mit einem Alkalimetallazid, z.B. mit Natriumazid, umsetzt, beispielsweise in Dimethylsulfoxid oder Dimethylformamid, oder indem man einen der Formel IV entsprechenden Alkohol (Z = Hydroxy) in Gegenwart von Triphenylphosphan und eines Azodicarbonsäureesters, z.B. von Azodicarbonsäurediäthylester, mit Stickstoffwasserstoffsäure zur Reaktion bringt, beispielsweise in Toluol.

In Ausgangsstoffen IV gemäss der Verfahrensvariante b) bedeutet reaktionsfähiges verestertes Hydroxy beispielsweise Halogen z.B. Chlor, Brom oder Iod, oder Sulfonyloxy, wie $C_1$-$C_4$-Alkansulfonyloxy oder gegebenenfalls substituiertes Benzolsulfonyloxy, wie Methan-, Aethan-, Benzol-, p-Toluol- oder p-Brombenzolsulfonyloxy, oder Fluorsulfonyloxy.

Salze von Verbindungen (V) sind beispielsweise Alkali- oder Erdalkalimetallsalze derselben, wie deren Natrium-, Kalium- oder Calciumsalze.

Die Umsetzung erfolgt in üblicher Weise, beispielsweise in Gegenwart eines basischen Kondensationsmittels oder vorteilhaft, indem man die Komponente der Formel (V) als Salz einsetzt, erforderlichenfalls unter Erwärmen, vorzugsweise in einem Lösungs- oder Verdünnungsmittel. Basische Kondensationsmittel sind beispielsweise mit der Komponente der Formel (V) salzbildende basische Kondensationsmittel, wie Alkalimetallalkoholate, z.B. Natriummethanolat oder Natriumäthanolat, oder Alkalimetall-oder Erdalkalimetallamide, z.B. Natriumamid oder Lithiumdiisopropylamid. Wie erwähnt, wird die Ueberführung der Komponente der Formel (V) in eines ihrer Salze vorteilhaft vorab durchgeführt, z.B. durch Umsetzung mit einer der genannten Basen. Lösungsmittel sind für die Durohführung der Umsetzung in Gegenwart eines Alkoholates vorzugsweise die entsprechenden Alkohole und bei der Durchführung in Gegenwart von Amiden beispielsweise aprotische organische Lösungsmittel, wie Phosphor-säurei -$C_1$-$C_4$-alkylamide, z.B. Hexamethylphosphorsäuretriamid, Alkansäureamide, z.B. Dimethylformamid, oder Di-$C_1$-$C_4$- alkylsulfoxide, z.B. Dimethylsulfoxid. Verfahrensgemäss als Nebenprodukt gebildete Isomere sind ggf. von den gewünschten Verbindungen der Formel (I) abzutrennen.

Die Ausgangsstoffe (IV) und (V) können, sofern sie nicht bereits bekannt sind, in üblicher Weise hergestellt werden. So erhält man Verbindungen der Formel (IV) z.B., indem man einen entsprechenden Alkohol (IV; Z = Hydroxy) reaktionsfähig verestert, z.B. mittels Thionylchlorid, Phosphortribromid oder eines Sulfonylchlorides. Verbindungen (V) können hergestellt werden, indem man Trimethylsilylazid oder Stickstoffwasserstoffsäure mit einer Verbindung der Formel

$$R_1-C\equiv C-R_2 \quad (IIIa) \quad bzw. \quad R_1-C(=O)-CH_2-R_2 \quad (IIIb),$$

worin $R_1$ insbesondere Carbamyl oder N,N-Di-C1-C4-alkylcarbamyl bedeutet, umsetzt und in einem gegebenenfalls erhaltenen 1-Trimethylsilyltriazolderivat die Silylgruppe, gewünschtenfalls nach N-Alk(ano)ylierung von Carbamyl $R_2$ und/oder $R_1$ wie für Verbindungen der Formel I nachstehend angegeben, durch milde Hydrolyse abspaltet. Man kann aber auch Trimethylsilylazid mit einer Verbindung der Formel

$$Y_5-C\equiv C-Y_4 \quad (VIIa) \quad bzw. \quad R_1-C(=O)-CH_2-Y_4 \quad (VIIb),$$

worin $Y_4$ einen in Carbamyl oder N,N-Di-$C_1$-$C_4$-alkylcarbamyl überführbaren Rest $Y_A$, beispielsweise verestertes Carboxy, wie $C_1$-$C_4$-Alkoxycarbonyl, oder Cyano, und $Y_5$ Wasserstoff oder vorzugsweise einen in Carbamyl oder N,N-Di-$C_1$-$C_4$-alkylcarbamyl überführbaren Rest $Y_B$, der vorzugsweise mit $Y_A$ identisch ist, bedeutet, umsetzen und $Y_A$ und/oder $Y_B$ in Carbamyl oder N,N-Di-$C_1$-$C_4$-alkylcarbamyl überführen, im Falle von verestertem Carboxy beispielsweise durch Ammonolyse (Umsetzung mit Ammoniak) bzw. im Falle von

Cyano beispielsweise durch Hydrolyse, wobei auch die Trimethylsilylgruppe abgespalten wird.

Gemäss der Verfahrensvariante c in Carbamylb oder N,N-Di-$C_1$-$C_4$-alkylcarbamyl überführbare Reste $Y_A$ und/oder $Y_B$ sind beispielsweise freie oder in einer Salz- oder Anhydridform vorliegende Carboxylgruppen, Amidinogruppen oder veresterte Carboxygruppen, ferner Gyanogruppen.

Veresterte Carboxygruppen sind beispielsweise mit einem $C_1$-$C_4$-Alkanol oder einem $C_1$-$C_4$-Alkylmercaptan veresterte Carboxygruppen, d.h. $C_1$-$C_4$-Alkoxy-oder $C_1$-$C_4$-Alkylthiocarbonylgruppen, können aber auch mit einem beliebigen anderen Alkohol oder Mercaptan, z.B. mit einem gegebenenfalls substituierten Phenol oder Thiophenol, verestert sein.

In Salzform vorliegende Carboxygruppen sind beispielsweise in einer von Ammoniak oder einem Di-$C_1$-$C_4$-alkylamin abgeleiteten Ammoniumsalzform vorliegende Carboxygruppen, ferner in Metall-, z.B. Alkali- oder Erdalkalimetallsalzform vorliegende Carboxygruppen.

In Anhydridform vorliegende Carboxygruppen sind beispielsweise in einer Halogenidform vorliegende Carboxygruppen, wie Chlorcarbonyl, können aber auch mit einer reaktiven Carbonsäure anhydridisiert sein und beispielsweise Alkoxycarbonyloxycarbonyl oder Trifluoracetoxycarbonyl bedeuten.

Die Ueberführung der genannten Gruppen $Y_A$ und/oder $Y_B$ in ggf. N,N-Di-$C_1$-$C_4$-alkyliertes Carbamyl erfolgt in üblicher Weise, ausgehend von freien, veresterten oder in einer Anhydridform vorliegenden Carboxygruppen und Amidinogruppen durch Solvolyse, d.h. Hydrolyse oder Ammonolyse bzw. Aminolyse( Umsetzung mit Ammoniak).

Durch Hydrolyse kann man beispielsweise Cyanogruppen oder Amidinogruppen $Y_A$ und/oder $Y_B$ in Carbamyl überführen. Die Hydrolyse von Cyanogruppen erfolgt beispielsweise in Gegenwart von basischen Hydrolysemitteln, wie Alkalimetallhydroxiden, z.B. Natrium- oder Kaliumhydroxid, erforderlichenfalls in Anwesenheit von Peroxyverbindungen, z.B. Wasserstoffperoxid. Die Hydrolyse von Amidinogruppen wird beispielsweise in Gegenwart eines sauren Hydrolysemittels, wie einer Mineral-, Sulfon- oder Carbonsäure, z.B. von Schwefelsäure, Phosphorsäure, Salzsäure oder einer anderen Halogenwasserstoffsäure, p-Toluolsulfonsäure oder einer anderen organischen Sulfonsäure, oder einer $C_1$-$C_4$-Alkansäure, wie Essigsäure, vorzugsweise in katalytischen Mengen, durchgeführt.

Durch Ammonolyse bzw. Aminolyse kann man beispielsweise freie oder in einer Salz- oder Anhydridform vorliegende oder veresterte Carboxygruppen in Carbamyl überführen. Dabei arbeitet man erforderlichenfalls in Gegenwart eines Kondensationsmittels, vorteilhaft in einem inerten Lösungsmittel. Kondensationsmittel sind basische Kondensationsmittel, vor allem Ammoniak bzw. für die Aminolyse verwendete Amine Ueberschuss, ausgehend von in Anhydridform vorliegendem Carboxy ferner Alkalimetallhydroxide oder -carbonate oder tertiäre organische Stick-stoffbasen, wie Tri-$C_1$-$C_4$-alkylamine oder tertiäre heteroaromatische Stickstoffbasen, wie Triäthylamin oder Pyridin. Freie Carboxygruppen können unter Dehydratisierung der intermediär gebildeten Ammoniumsalze, z.B. durch Erhitzen oder Einwirkung wasserentziehender Mittel, wie von Säureanhydriden, z.B. Phosphorpentoxid und dergleichen, oder von Carbodiimiden, z.B. N,N'-Dicyclohexylcarbodiimid, in Carbamyl überführt werden. Eine besonders bevorzugte Ausführungsform dieser Verfahrensvariante ist dadurch gekennzeichnet, dass man eine Verbindung der Formel VI, wori $Y_4$ verestertes Carboxy bedeutet und $Y_5$ Wasserstoff oder ebenfalls verestertes Carboxy darstellt, mit einem Ueberschuss an Ammoniak oder an einem Di-$C_1$-$C_4$-alkylamin umsetzt.

Die Ausgangsstoffe (VI) können, soweit sie nicht bekannt sind, in üblicher Weise hergestellt werden, beispielsweise, indem man ein Azid der Formel

$$Ph - alk - N_3 \qquad (II)$$

mit einer Verbindung der Formel

$$Y_5-C{\equiv}C-Y_4 \quad (VIIa) \qquad bzw. \qquad R_1-C(=O)-CH_2-Y_4 \quad (VIIb)$$

umsetzt, beispielsweise in analoger Weise wie unter der Verfahrens-variante a) beschrieben. Erforderlichenfalls können primär erhaltene Ester bzw. Nitrile der Formel VI ($Y_4$ und ggf. $Y_5$ = verestertes Carboxy oder Cyano) unter basischen Bedingungen, z.B. mittels wässrig-alkoholischer Natronlauge, zur entsprechenden Säure hydrolysiert und primär oder durch Hydrolyse entsprechender Ester oder Nitrile erhaltene Säuren der Formel VI ($Y_4$ und ggf. $Y_5$ = Carboxy), z.B. mit Thionylchlorid, in die Säurechloride überführt werden.

In verfahrensgemäß erhältlichen Verbindungen der Formel (I) kann man durch Behandeln mit einem Alkanoylierungsmittel N-unsubstituiertes Carbamyl in N-$C_2$-$C_5$-Alkanoylcarbamyl überführen.

Alkanoylierungsmittel sind beispielsweise $C_1$-$C_4$-Alkancarbosäureanhydride, wie Acetanhydrid oder das gemischte Anhydrid von Ameisen- und Essigsäure, bzw. $C_1$-$C_4$-Alkancarbonsäurechloride, wie Acetylchlorid.

Die Umsetzung mit diesen erfolgt in üblicher Weise, erforderlichenfalls in Gegenwart einer Base, z.B. von Triäthylamin oder Pyridin, bzw. im Falle der Umsetzung mit Säureanhydriden in Gegenwart einer Mineralsäure, z.B. von Schwefelsäure.

Die Auftrennung von Isomerengemischen, als welche beispielsweise Enantiomeren-bzw. Diastereomerengemische von Verbindungen der Formel (I) mit mindestens einem asymmetrischen C-Atom sowie Gemische von Verbindungen der Formel (I) und verfahrensgemäss gebildeten Isomeren derselben zu nennen sind, erfolgt in üblicher Weise. Diastereoisomerengemische sowie Gemische von Verbindungen der Formel (I) und verfahrensgemäss gebildeten Isomeren derselben können beispielsweise auf Grund der unterschiedlichen physikalischen Eigenschaften der Komponenten durch übliche physikalische Trennverfahren, z.B. durch fraktionierte Kristallisation, Chromatographieverfahren und dergleichen, getrennt werden. Für die Auftrennung von Enantiomerengemischen ist beispielsweise die fraktionierte Kristallisation aus einem optisch aktiven Lösungsmittel oder die Chromatographie an einer optisch aktiven stationären und/oder mobilen Phase geeignet. Man kann aber ein Enantiomerengemisch auch, z.B. durch Umsetzung mit einem optisch aktiven Säurechlorid, in die entsprechenden diastereomeren Acylderivate überführen, diese in die Komponenten auftrennen und aus diesen, z.B. durch milde Säurebehandlung, die reinen Enantiomeren freisetzen.

Die Verbindungen der Formel (I) können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Träger-stoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Laktose, Dextrose, Saccharose, Mannitol, Sorbitol, Cellulose und/oder Glycin, und/oder Schmiermittel, z.B. Kieselerde, Talk, Stearinsäure oder Salze davon, wie Magnesium-oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxylmethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmack-stoffe und Süssmittel aufweisen. Ferner kann man die Verbindungen der Formel (I) in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 %, des Aktivstoffes.

Die Erfindung betrifft ebenfalls die Verwendung der Verbindungen der Formel (I), vorzugsweise in Form von pharmazeutischen Präparaten. Die Dosierung kann von verschiedenen Faktoren, wie Applikationsweise, Spezies, Alter und/oder individuellem Zustand, abhängen. Die täglich zu verabreichenden Dosen liegen bei oraler Applikation zwischen etwa 1 und etwa 50 mg/kg in Einzeldosen von je etwa 1 bis etwa 25 mg/kg und für Warmblüter mit einem Gewicht von etwa 70 kg vorzugsweise in Tagesdosen von etwa 0,070 g bis etwa 3,5 g.

Die folgenden Beispiele dienen zur Illustration der Erfindung; Temperaturen werden in Celsiusgraden angegeben.

Beispiel 1: 73,5 g (0,25 Mol) 1-(o-Fluorbenzyl)-1H-1,2,3-triazol-4,5-dicarbonsäuredimethylester werden in 1000 ml Methanol gelöst. Dann presst man im Autoklaven 250 g Ammoniak auf und belässt 24 h bei 100°. Dann wird abgekühlt, das auskristallisierte Produkt abgesaugt, mit Methanol gewaschen und aus Dioxan/Toluol umkristallisiert. Man erhält das 1-(o-Fluorbenzyl)-1H-1,2,3-triazol-4,5-dicarboxamid vom Smp. 197-199°.

Das Ausgangsmaterial kann z.B. folgendermassen hergestellt werden: Zu einer auf 90° erwärmten Lösung von 41,5 g (0,275 Mol) o-Fluorbenzyl-azid in 50 ml Toluol wird eine Lösung von 40 g (0,282 Mol) Acetylendicarbonsäuredimethylester in 500 ml Toluol zugetropft. Nach 5 weiteren Stunden bei 90° zieht man das Toluol ab und saugt nach Abkühlen die auskristallisierte Substanz ab. Nach Umkristallisieren aus einem Gemisch von Diäthyläther und Petroläther (1:1) erhält man so den 1-(o-Fluor-benzyl)-1H-1,2,3-triazol-4,5-dicarbonsäuredimethylester vom Smp. 49-51°.

Beispiel 2: 59 g (0,26 Mol) 1-(o-Fluorbenzyl)-1H-1,2,3-triazol-4-carbonsäure werden mit 300 ml Thionylchlorid 1 Stunde zum Rückfluss erhitzt. Dann destilliert man das überschüssige Thionylchlorid im Vakuum, löst das zurückbleibende 1-(o-Fluorbenzyl)-1H-1,2,3-triazol-4-carbonsäurechlorid in 500 ml Toluol und tropft diese Lösung bei 5-10° zu 500 ml einer konzentrierten wässrigen Ammoniaklösung. Das ausgefallene Produkt wird abgesaugt, mit Wasser gewaschen und aus Aethanol umkristallisiert. Man erhält so das 1-(o-Fluorbenzyl)-1H-1,2,3-triazol-4-carboxamid vom Smp. 220-222°.

Das Ausgangsmaterial kann z.B. folgendermassen hergestellt werden: Eine Lösung von 50 g (0,33 Mol)-o-Fluorbenzylazid; 23,1 g (0,33 Mol) Propincarbonsäure und 400 ml Toluol wird 24 Stunden bei 70° gerührt. Das ausgefallene Produkt wird nach dem Abkühlen auf Raumtemperatur abgesaugt und zunächst mit Toluol und dann mit Diäthyläther gewaschen. Man erhält so die 1-(o-Fluorbenzyl)-1H-1,2,3-triazol-4-carbonsäure vom Smp. 151° (Zers.).

Beispiel 3: In analoger Weise wie in Beispiel 1 beschriebene erhält man, ausgehend von 2,6-Difluorbenzylazid und Acetylendicarbonsäuredimethylester, über den 1-(2,6-Difluorbenzyl)-1H-1,2,3-triazol-4,5-dicarbonsäuredimethylester (Smp. 62-65°), das 1-(2,6-Difluorbenzyl)-1H-1,2,3-triazol-4,5-dicarboxamid vom Smp. 203-205° (aus Methanol).

Beispiel 4: In analoger Weise wie in Beispiel 2 beschrieben erhält man, ausgehend von 2,6-Difluorbenzylazid, über 1-(2,6-Difluorbenzyl)-1H-1,2,3-triazol-4-carbonsäure vom Smp. 160-162° (aus Acetonitril; Zers.) das 1-(2,6-Difluorbenzyl)-1H-1,2,3-triazol-4-carboxamid vom Smp. 237-240° (aus Aethanol).

Beispiel 5: In analoger Weise wie in den Beispielen 1-4 beschrieben kann man ferner herstellen:
1-(2, 3-Difluorbenzyl)-1H-1,2,3-triazol-4-carboxamid,
1-(2,4-Difluorbenzyl)-1H-1,2,3-triazol-4-carboxamid und
1-(2,5-Difluorbenzyl)-1H-1,2,3-triazol-4-carboxamid.

Beispiel 6: In analoger Weise wie in Beispiel 2 beschrieben erhält man aus 1-(6-Chlor-2-fluor-benzyl)-1H-1,2,3-triazol-4-carbonsäure das 1-(6-Chlor-2-fluor-benzyl)-1H-1,2,3-triazol-4-carboxamid vom Smp. 274-276° (aus Eisessig).

Das Ausgangsmaterial kann z.B. folgendermassen hergestellt werden: Eine Mischung von 98 g (0,678 Mol) 6-Chlor-2-fluor-toluol, 91,5 g (0,678 Mol) Sulfurylchlorid und 0,2 g Dibenzoylperoxyd wird 3 Stunden bei 100-110° gerührt und anschliessend destilliert. Man erhält so das 6-Chlor-2-fluorbenzylchlorid ($Kp_{15}$ = 78-82°).

Zu einer Suspension von 47 g (0`722 Mol) Natriumazid in 400 ml Dimethylsulfoxid tropft man bei 20-40° 123 g (0,687 Mol) 6-Chlor-2-fluor-benzyl-chlorid hinzu. Nach 4-stündigem Rühren bei Raumtemperatur wird mit 1 Liter Eiswasser verdünnt und mit Cyclohexan extrahiert. Nach Abdestillieren des Lösungsmittels wird der Rückstand destilliert. Man erhält so das 6-Chlor-2-fluorbenzylazid ($Kp_{15}$ = 99° – 100°).

27,5 g (0,15 Mol) 6-Chlor-2-fluor-benzylazid und 10,5 g (0,15 Mol) Propincarbonsäure in 300 ml Toluol erhitzt man 3 Stunden auf 90°. Nach Abkühlen saugt man die Kristalle ab und kristallisiert aus Acetonitril um. Man erhält so die 1-(6-Chlor-2-fluor-benzyl)-1H-1,2,3-triazol-4-carbonsäure vom Smp. 182° (Zers.).

Beispiel 7: In analoger Weise wie in Beispiel 1 beschrieben erhält man, ausgehend von 6-Ghlor-2-fluorbenzylazid und Acetylendicarbonsäuredimethylester, über den 1-(6-Chlor-2-fluor-benzyl)-1H-1,2,3-triazol-4,5-dicarbonsäuredimethylester (Smp. 88-90°) das 1-(6-Chlor-2-fluor-benzyl)-1H-1,2,3-triazol-4,5-dicarboxamid vom Smp. 214-216° (aus Eisessig).

Beispiel 8: In analoger Weise wie in Beispiel 1 beschrieben erhält man aus 2,5-Difluorbenzylazid ($Kp_{15}$ = 82-84°) über 1-(2,5-Difluorbenzyl)-1H-1,2,3-triazol-4,5-dicarbonsäuredimethylester das 1-(2,5-Difluor-benzyl)-1H-1,2,3-triazol-4,5-dicarboxamid vom Smp. 191-192° (aus Dioxan/Toluol).

Beispiel 9: In analoger Weise wie in Beispiel 1 beschrieben erhält man aus 2,4-Difluorbenzylazid ($Kp_{15}$ = 80-83°) über den 1-(2,4-Difluorbenzyl)-1H-1,2,3-triazol-4,5-dicarbonsäuredimethylester vom Smp. 75-76° (aus Cyclohexan) das 1(2,4-Difluorbenzyl)-1H-1,2,3-triazol-4,5-dicarboamid vom Smp. 183-185° (aus Dioxan/Toluol).

Beispiel 10: Analog Beispiel 1 erhält man durch Umsetzung mit Dimethylamin das 1-(2,6-Difluorbenzyl)-1H-1,2,3-triazol-4,5-dicarbonsäuredi(N,N-dimethyl)amid vom Smp. 130-133° (aus Tertiärbutoxymethan).

Beispiel 11: Analog Beispiel 1 erhält man aus 2,3-Difluorbenzylazid und Acetylendicarbonsäuredimethylester über 1-(2,3-Difluorbenzyl)-1H-1,2,3-triazol-4,5-dicarbonsäuredimethylester das 1-(2,3-Difluorbenzyl)-1H-1,2,3-triazol-4,5-dicarboamid vom Smp. 183-185° (aus Essigester/Benzol).

Beispiel 12: 2,81 g (10 Mol) 1-( 2,6-Difluorbenzyl)-1H-1,2,3-triazol-4,5-dicarboamid, 20 ml Acetanhydrid und 2 Tropfen Schwefelsäure werden 3 Stunden auf 80° erhitzt. Nach dem Abkühlen verrührt man mit 100 ml Wasser 1 Stunde bei 20-25°, saugt das ausgefallene Produkt ab und wäscht mit Wasser. Nach Umkristallisation aus 75 ml Methanol erhält man so das 1-(2,6-Difluorbenzyl)-1H-1,2,3-triazol-4,5-di(N-acetyl)carboxamid vom Smp 136-138°.

Beispiel 13: In analoger Weise wie in Beispiel 12 beschrieben erhält man auch das 1-(2,6-Difluorbenzyl)-1H-1,2,3-triazol-4-(N-acetyl)-carboxamid vom Smp. 205-207° (aus Dioxan-Toluol).

Beispiel 14: 1,4 g (12,5 mMol) Acetylendicarbonsäurediamid werden bei 60° in 10 ml Dimethylsulfoxid gelöst. Man gibt 1,69 g (10 mMol) 2,6-Difluorbenzylazid hinzu und lässt 16 Stunden bei 80° rühren. Dann verdünnt man mit 25 ml Wasser, saugt ab, verrührt mit 50 ml Wasser, lässt 30 Minuten bei 50-60° rühren, saugt erneut ab, trocknet und kristallisiert aus Dioxan/Aethanol um. Man erhält das 1-(2,6-Difluorbenzyl)-1H-1,2,3-triazol-4,5-dicarboxamid vom Smp. 203-205°.

Beispiel 15: Tabletten, enthaltend je 50 mg 1-(o-Fluorbenzyl)-1H-1,2,3-triazol-4-carboxamid, können wie folgt hergestellt werden

| Zusammensetzung (10'000 Tabletten) | |
|---|---|
| Wirkstoff | 500.0 g |
| Lactose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdisper.) | 20.0 g |
| Aethanol | q.s |

: Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiele 16: Lacktabletten, enthaltend je 100 mg 1-(o-Fluorbenzyl)-1H-1,2,3-triazol-4-carboxamid, können wie folgt hergestellt werden:

| Zusammensetzung (für 1000 Tabletten) | |
|---|---|
| Wirkstoff | 100.00 g |
| Lactose | 100.00 g |
| Maisstärke | 70.00 g |
| Talk | 8.50 g |
| Calciumstearat | 1.50 g |
| Hydroxypropyl-methylcellulose | 2.36 |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; das Endgewicht der Lacktablette beträgt 283 mg.

Beispiel 17: In analoger Weise wie in den Beispielen 15 und 16 beschrieben, können auch Tabletten bzw. Lacktabletten enthaltend eine andere Verbindung gemäss einem der Beispiele 1-14 hergestellt werden.

## Ansprüche

1. Fluorierte 1-(α-Phenylalkyl)-1H-1,2,3-triazolverbindungen der Formel

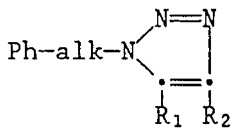

(I),

worin Ph in o-Stellung durch Fluor und gegebenenfalls zusätzlich durch bis und mit 2 Chloratome, 1 Fluor- und 1 Chloratom oder bis und mit 2 Fluoratome substituiertes Phenyl bedeutet, alk für Methylen steht , $R_1$ Wasserstoff, Carbamyl, N-$C_2$-$C_5$-Alkanoylcarbamyl oder N,N-Di-$C_1$-$C_4$-Alkylcarbamyl ist und $R_2$ Carbamyl, N-$C_2$-$C_5$-Alkanoylcarbamyl oder N,N-Di-$C_1$-$C_4$-alkylcarbamyl darstellt.

2. Verbindungen gemäss Anspruch 1, worin Ph in o-Stellung durch Fluor und gegebenenfalls zusätzlich durch 1 Chloratom, 1 Fluor- und 1 Chloratom oder bis und mit 2 Fluoratome substituiertes Phenyl bedeutet, alk für Methylen steht, $R_1$ Wasserstoff oder einen Rest $R_2$ bedeutet und $R_2$ Carbamyl oder N,N-Di-$C_1$-$C_4$-alkylcarbamyl darstellt.

3. Verbindungen gemäss Anspruch 1, worin Ph o-Fluorphenyl, 2,3-, 2,4-, 2,5- oder 2,6-Difluorphenyl oder 6-Chlor-2-fluor-phenyl bedeutet, alk für Methylen steht, $R_1$ Wasserstoff oder Carbamyl bedeutet und $R_2$ Carbamyl darstellt.

4. Verbindungen gemäss Anspruch 1, worin Ph o-Fluorphenyl oder 2,6-Di-fluorphenyl bedeutet, alk Methylen darstellt, $R_1$ Wasserstoff oder Carbamyl bedeutet und $R_2$ Carbamyl darstellt.

5. Verbindungen gemäss Anspruch 1, worin Ph 2,6-Difluorphenyl bedeutet, alk Methylen darstellt, $R_1$ Wasserstoff oder einen Rest $R_2$ bedeutet und $R_2$ für Carbamyl, N-$C_2$-$C_5$-Alkanoylcarbamyl oder N,N-Di-$C_1$-$C_4$-alkylcarbamyl steht.

6. Verbindungen gemäss Anspruch 1, worin Ph o-Fluorphenyl, 2,5-Difluorphenyl, 2,6-Difluorphenyl oder 2-Chlor-6-fluor-phenyl bedeutet, alk für Methylen steht und $R_1$ und $R_2$ beide Carbamyl darstellen.

7. Verbindungen gemäss Anspruch 1, worin Ph 2,6-Difluorphenyl, alk Methylen, $R_1$ Wasserstoff oder Carbamyl und $R_2$ Carbamyl darstellt.

8. 1-(o-Fluorbenzyl)-1H-1,2,3-triazol-4-carboxamid gemäss Anspruch 1.

9. 1-(2,6-Difluorbenzyl)-1H-1,2,3-triazol-4-carboxamid gemäss Anspruch 1.

10. 1-(o-Fluorbenzyl)-1H-1,2,3-triazol-4,5-dicarboxamid gemäss Anspruch 1.

11. 1-(2,6-Difluorbenzyl)-1H-1,2,3-triazol-4,5-dicarboxamid gemäss Anspruch 1.

12. 1-(6-Chlor-2-fluor-benzyl)-1H-1,2,3-triazol-4-carboxamid gemäss Anspruch 1.

13. 1-(6-Chlor-2-fluor-benzyl)-1H-1,2,3-triazol-4,5-dicarboxamid gemäss Anspruch 1.

14. 1-(2,5-Difluorbenzyl)-1H-1,2,3-triazol-4,5-dicarboxamid gemäss Anspruch 1.

15. 1-(2,6-Difluorbenzyl)-1H-1,2,3-triazol-4,5-di(N,N-dimethyl)-carboxamid gemäss Anspruch 1.

16. 1-(2,6-Difluorbenzyl)-1H-1,2,3-triazol-4-(N-acetyl)carboxamid gemäss Anspruch 1.

17. 1-(2,6-Difluorbenzyl)-1H-1,2,3-triazol-4,5-di(N-acetyl)carboxamid gemäss Anspruch 1.

18. 1-(2,3-Difluorbenzyl)-1H-1,2,3-triazol-4-carboxamid gemäss Anspruch 1.

**19.** 1(2,3-Difluorbenzyl)-1H-1,2,3-triazol-4,5-dicarboxamid gemäss Anspruch 1.

**20.** 1-(2,4-Difluorbenzyl)-1H-1,2,3-triazol-4,5-dicarboxamid gemäss Anspruch 1.

**21.** Eine Verbindung gemäss einem der Ansprüche 1, 2, 5 und 15 bis 20 zur Anwendung in einem Verfahren zur therapeutischen Behandlung des menschlichen oder tierischen Körpers, beispielsweise als antikonvulsives Mittel.

**22.** Eine Verbindung gemäss einem der Ansprüche 3, 4 und 6 bis 14 zur Anwendung in einem Verfahren zur therapeutischen Behandlung des menschlichen oder tierischen Körpers, beispielsweise als antikonvulsives Mittel.

**23.** Pharmazeutische Präparate, enthaltend eine Verbindung gemäss einem der Ansprüche 1, 2, 5 und 15 bis 21 neben üblichen pharmazeutischen Hilfs- und/oder Trägerstoffen.

**24.** Pharmazeutische Präparate, enthaltend eine Verbindung gemäss einem der Ansprüche 3, 4, 6 bis 14 und 22 neben üblichen pharmazeutischen Hilfs- und/oder Trägerstoffen.

**25.** Verfahren zur Herstellung von Verbindungen der formel I gemäss Anspruch 1, dadurch gekennzeichnet, dass man a) eine Verbindung der formel

$$\overline{Ph} - alk - N_3 \qquad (II)$$

mit einer Verbindung der Formel

$$R_1 - \underset{Y_1}{C} = \underset{Y_2}{C} - R_2 \qquad (III),$$

worin $Y_1$ Hydroxy und $Y_2$ Wasserstoff darstellt oder $Y_1$ und $Y_2$ gemeinsam eine zusätzliche Bindung bedeuten, oder einem Salz und/oder Tautomeren davon umsetzt oder b) eine Verbindung der Formel

$$Ph - alk - Z \qquad (IV),$$

worin Z reaktionsfähiges verestertes Hydroxy bedeutet, mit einem 1H-1,2,3-Triazolderivat der Formel

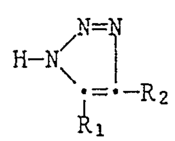

(V)

oder einem Salz davon umsetzt oder c) Zur Herstellung von Verbindungen der Formel I, worin entweder $R_1$ Wasserstoff, Carbamyl, N-$C_2$-$C_5$-Alkanoylcarbamyl oder N,N-Di-$C_1$-$C_4$-alkylcarbamyl ist und $R_2$ Carbamyl oder N,N-Di-$C_1$-$C_4$-alkylcarbamyl ist oder $R_1$ Carbamyl oder N,N-Di-$C_1$-$C_4$ alkylcarbamyl ist und $R_2$ Carbamyl, N-$C_2$-$C_5$-Alkanoylcarbamyl oder N,N-Di-$C_1$-$C_4$-alkylcarbamyl ist, in einer Verbindung der Formel

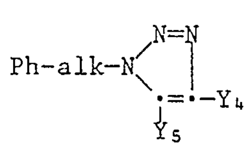

(VI),

worin $Y_4$ einen in Carbamyl oder N,N-Di-$C_1$-$C_4$-alkylcarbamyl überführbaren Rest $Y_A$ und $Y_5$ einen Rest $R_1$ oder einen in Carbamyl oder N,N-Di-$C_1$-$C_4$-alkylcarbamyl überführbaren Rest $Y_B$ oder $Y_4$ einen Rest $R_2$ und $Y_5$ einen in Carbamyl oder N,N-Di-$C_1$-$C_4$-alkylcarbamyl überführbaren Rest $Y_B$ darstellt, $Y_A$

und/oder $Y_B$ in Carbamyl oder N,N-Di-$C_1$-$C_4$-alkylcarbamyl überführt, erforderlichenfalls ein erhaltenes Isomerengemisch in die Komponenten auftrennt und das Isomere der Formel (I) isoliert und gewünschtenfalls eine Verfahrensgemäss erhältliche Verbindung der Formel I in eine andere Verbindung der Formel (I) umwandelt und/oder ein verfahrensgemäss erhältliches Enantiomeren- bzw. Diastereomerengemisch in die Komponenten aufspaltet.

**26.** Verwendung einer Verbindung gemäss einem der Ansprüche 1-22 zur Herstellung eines antikonvulsiven Arzneimittels.

Patentansprüche für folgenden Vertragsstaat : AT

**1.** Verfahren zur Herstellung fluorierter 1-(α-Phenylalkyl)-1H-1,2,3-triazolverbindungen der Formel

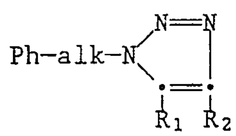 (I),

worin Ph in o-Stellung durch Fluor und gegebenenfalls zusätzlich durch bis und mit 2 Chloratome, 1 Fluor- und 1 Chloratom oder bis und mit 2 Fluoratome substituiertes Phenyl bedeutet, alk für Methylen steht, $R_1$ Wasserstoff, Carbamyl, N-$C_2$-$C_5$-Alkanoylcarbamyl oder N,N-Di-$C_1$-$C_4$-alkylcarbamyl ist und $R_2$ Carbamyl, N-$C_2$-$C_5$-Alkanoylcarbamyl oder N,N-Di$C_1$-$C_4$-alkylcarbamyl darstellt, dadurch gekennzeichnet, dass man a) eine Verbindung der Formel

$$Ph - alk - N_3 \qquad (II)$$

mit einer Verbindung der Formel

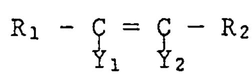 (III),

worin $Y_1$ Hydroxy und $Y_2$ Wasserstoff darstellt oder $Y_1$ und $Y_2$ gemeinsam eine zusätzliche Bindung bedeuten, oder einem Salz und/oder Tautomeren davon umsetzt oder b) eine Verbindung der Formel

$$Ph - alk - Z \qquad (IV),$$

worin 2 reaktionsfähiges verestertes Hydroxy bedeutet, mit einem 1H-1,2,3-Triazolderivat der Formel

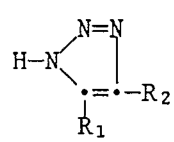 (V)

oder einem Salz davon umsetzt oder c) Zur Herstellung von Verbindungen der Formel I, worin entweder $R_1$ Wasserstoff, Carbamyl, N-$C_2$-$C_5$-Alkanoylcarbamyl oder N,N-Di-$C_1$-$C_4$-alkylcarbamyl ist und $R_2$ Carbamyl oder N,N-Di-$C_1$-$C_4$-alkylcarbamyl ist oder $R_1$ Carbamyl oder N,N-Di-$C_1$-$C_4$ alkylcarbamyl ist und $R_2$ Carbamyl, N-$C_2$-$C_5$-Alkanoylcarbamyl oder N,N-Di-$C_1$-$C_4$-alkylcarbamyl ist, in einer Verbindung der Formel

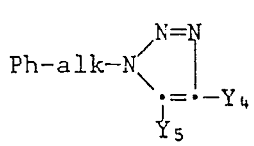 (VI),

worin $Y_4$ einen in Carbamyl oder N,N-Di-$C_1$-$C_4$-alkylcarbamyl überführbaren Rest $R_A$ und $Y_5$ einen Rest $R_1$ oder einen in Carbamyl oder N,N-Di-$C_1$-$C_4$-alkylcarbamyl überführbaren Rest $Y_B$ oder $Y_4$ einen Rest $R_2$ und $Y_5$ einen in Caramyl oder N,N-Di-$C_1$-$C_4$-alkylcarbamyl überführbaren Rest $Y_B$ darstellt, $Y_A$ und/oder $Y_B$ in Carbamyl oder N,N-Di-$C_1$-$C_4$-alkylcarbamyl überführt, erforderlichenfalls ein erhaltenes Isomerengemisch in die Komponenten auftrennt und das Isomere der Formel (I) isoliert und gewünschtenfalls eine verfahrensgemäss erhältliche Verbindung der Formel I in eine andere Verbindung der Formel (I) umwandelt und/oder ein verfahrensgemäss erhältliches Enantiomeren- bzw. Diastereomerengemisch in die Komponenten aufspaltet.

2. Verfahren gemäss Anspruch 1, dadurch gekennzeichnet, dass man eine Verbindung der Formel VI, worin $Y_4$ verestertes Carboxy bedeutet und $Y_5$ Wasserstoff oder ebenfalls verestertes Carboxy darstellt, mit einem Ueberschuss an Ammoniak oder an einem Di-$C_1$-$C_4$-alkylamin umsetzt

3. Verfahren gemäss Anspruch 1 oder 2, dadurch gekennzeichnet, dass man Verbindungen der Formel (I), worin Ph in o-Stellung durch Fluor und gegebenenfalls zusätzlich durch 1 Chloratom, 1 Fluor- und 1 Chloratom oder bis und mit 2 Fluoratome substituiertes Phenyl bedeutet, alk für Methylen steht, $R_1$ Wasserstoff oder einen Rest $R_2$ bedeutet und $R_2$ Carbamyl oder N,N-Di-$C_1$-$C_4$-al kylcarbamyl darstellt, herstellt.

4. Verfahren gemäss Anspruch 1 oder 2, dadurch gekennzeichnet, dass man Verbindungen der Formel (I), worin Ph o-Fluorphenyl, 2,3-, 2,4-, 2,5-oder 2,6-Difluorphenyl oder 6-Chlor-2-fluor-phenyl bedeutet, alk für Methylen steht, $R_1$ Wasserstoff oder Carbamyl bedeutet und $R_2$ Carbamyl darstellt, herstellt.

5. Verfahren gemäss Anspruch 1 oder 2, dadurch gekennzeichnet, dass man Verbindungen der Formel (I), worin Ph o-Fluorphenyl oder 2,6-Difluor-phenyl bedeutet, alk Hethylen darstellt, $R_1$ Wasserstoff oder Carbamyl bedeutet und $R_2$ Carbamyl darstellt, herstellt.

6. Verfahren gemäss Anspruch 1 oder 2, dadurch gekennzeichnet, dass man Verbindungen der Formel I, worin Ph o-Fluorphenyl, 2,5-Difluorphenyl, 2,6-Difluorphenyl oder 6-Chlor-2-fluor-phenyl bedeutet, alk für Methylen steht und $R_1$ und $R_2$ beide Carbamyl darstellen, herstellt.

7. Verfahren gemäss Anspruch 1 oder 2, dadurch gekennzeichnet, dass man Verbindungen der Formel I, worin Ph 2,6-Difluorphenyl, alk Methylen, $R_1$ Wasserstoff oder Carbamyl und $R_2$ Carbamyl darstellt, herstellt.

8. Verfahren gemäss Anspruch 1 oder 2, dadurch gekennzeichnet, dass man 1-(o-Fluorbenzyl)-1H-1,2,3-triazol-4-carboxamid herstellt.

9. Verfahren gemäss Anspruch 1 oder 2, dadurch gekennzeichnet, dass man 1-(2,6-Difluorbenzyl)-1H-1,2,3-triazol-4-carboxamid herstellt.

10. Verfahren gemäss Anspruch 1 oder 2, dadurch gekennzeichnet, dass man 1-(o-Fluorbenzyl)-1H-1,2,3-triazol-4,5-dicarboxamid herstellt.

11. Verfahren gemäss Anspruch 1 oder 2, dadurch gekennzeichnet, dass man 1-(2,6-Difluorbenzyl)-1H-1,2,3-triazol-4,5-dicarboxamid herstellt.

12. Verfahren gemäss Anspruch 1 oder 2, dadurch gekennzeichnet, dass man 1-(6-Chlor-2-fluor-benzyl)-1H-1,2,3-triazol-4-carboxamid herstellt.

13. Verfahren gemäss Anspruch 1 oder 2, dadurch gekennzeichnet, dass man 1-(6-Chlor-2-fluor-benzyl)-1H-1,2,3-triazol-4,5-dicarboxamid herstellt.

14. Verfahren gemäss Anspruch 1 oder 2, dadurch gekennzeichnet, dass man 1-(2,5-Difluorbenzyl)-1H-1,2,3-triazol-4,5-dicarboxamid herstellt.

15. Verfahren gemäss Anspruch 1 oder 2, dadurch gekennzeichnet, dass man 1-(2,6-Difluorbenzyl)-1H-1,2,3-triazol 4,5-di(N,N-dimethyl) carboxamid herstellt.

16. Verfahren gemäss Anspruch 1 oder 2, dadurch gekennzeichnet, dass man 1-(2,6-Difluorbenzyl)-1H-1,2,3-triazol-4-(N-acetyl)carboxamid herstellt.

17. Verfahren gemäss Anspruch 1 oder 2, dadurch gekennzeichnet, dass man 1-(2,6-Difluorbenzyl)-1H-1,2,3-triazol-4,5-di(N-acetyl)-carboxaid herstellt

18. Verfahren gemäss Anspruch 1 oder 2, dadurch gekennzeichnet, dass man 1-(2,3-Difluorbenzyl)-1H-1,2,3-triazol-4-carboxamid herstellt

19. Verfahren gemäss Anspruch 1 oder 2, dadurch gekennzeichnet, dass man 1(2,3-Difluorbenzyl)-1H-1,2,3-triazol-4,5-dicarboxamid herstellt.

20. Verfahren gemäss Anspruch 1 oder 2, dadurch gekennzeichnet, dass man 1(2,4-Difluorbenzyl)-1H-1,2,3-triazol-4,5-dicarboxamid herstellt.

21. Verfahren zur Herstellung pharmazeutischer Präparate, dadurch gekennzeichnet, dass man eine Verbindung der Formel I erhältlich gemäss einem der Ansprüche 1 - 20, mit üblichen pharmazeutischen Hilfs- und/oder Trägerstoffen vermischt.

## Claims

1. Fluorinated 1-(α-phenylalkyl)-1H-1,2,3-triazole compounds of the formula

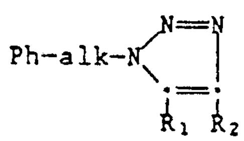

(I),

wherein Ph is an o-fluorinated phenyl radical which may be additionally substituted by up to 2 chlorine atoms inclusive, by 1 fluorine atom and 1 chlorine atom, or by up to 2 fluorine atoms inclusive, alk is methylene, $R_1$ is hydrogen, carbamoyl, N-($C_2$-$C_5$)alkanoylcarbamoyl or N,N-di($C_1$-$C_4$)alkylcarbamoyl, and $R_2$ is carbamoyl, N-($C_2$-$C_5$)alkanoylcarbamoyl or N,N-di($C_1$-$C_4$)alkylcarbamoyl.

2. Compounds according to claim 1, wherein Ph is an o-fluorinated phenyl radical which may be additionally substituted by 1 chlorine atom, by 1 fluorine atom and 1 chlorine atom, or by up to 2 fluorine atoms inclusive, alk is methylene, $R_1$ is hydrogen or a radical $R_2$, and $R_2$ is carbamoyl or N,N-di($C_1$-$C_4$)alkylcarbamoyl.

3. Compounds according to claim 1, wherein Ph is o-fluorophenyl, 2,3-, 2,4-, 2,5- or 2,6-difluorophenyl or 6-chloro-2-fluorophenyl, alk is methylene, $R_1$ is hydrogen or carbamoyl and $R_2$ is carbamoyl.

4. Compounds according to claim 1, wherein Ph is ofluorophenyl or 2,6-difluorophenyl, alk is methylene, $R_1$ is hydrogen or carbamoyl and $R_2$ is carbamoyl.

5. Compounds according to claim 1, wherein Ph is 2,6-difluorophenyl, alk is methylene, $R_1$ is hydrogen or a radical $R_2$, and $R_2$ is carbamoyl, N-($C_2$-$C_5$)alkanoyl-carbamoyl or N,N-di($C_1$-$C_4$)alkylcarbamoyl.

6. Compounds according to claim 1, wherein Ph is o-fluorophenyl, 2,5-difluorophenyl, 2,6-difluorophenyl or 2-chloro-6-fluorophenyl, alk is methylene and $R_1$ and $R_2$ are both carbamoyl.

7. Compounds according to claim 1, wherein Ph is 2,6-difluorophenyl, alk is methylene, $R_1$ is hydrogen or carbamoyl and $R_2$ is carbamoyl.

8. 1-(o-Fluorobenzyl)-1H-1,2,3-triazole-4-carboxamide according to claim 1.

9. 1-(2,6-Difluorobenzyl)-1H-1,2,3-triazole-4-carboxamide according to claim 1.

**10.** 1-(o-Fluorobenzyl)-1H-1,2,3-triazole-4,5-dicarboxamide according to claim 1.

**11.** 1-(2,6-Difluorobenzyl)-1H-1,2,3-triazole-4,5-dicarboxamide according to claim 1.

**12.** 1-(6-Chloro-2-fluorobenzyl)-1H-1,2,3-triazole-4-carboxamide according to claim 1.

**13.** 1-(6-Chloro-2-fluorobenzyl)1H-1,2,3-triazole-4,5-dicarboxamide according to claim 1.

**14.** 1-(2,5-Difluorobenzyl)-1H-1,2,3-triazole-4,5-di-carboxamide according to claim 1.

**15.** 1-(2,6-Difluorobenzyl)-1H-1,2,3-triazole-4,5-di-(N,N-dimethyl)carboxamide according to claim 1.

**16.** 1-(2,6-Difluorobenzyl)-1H-1,2,3-triazole-4-(N-acetyl)carboxamide according to claim 1.

**17.** 1-(2,6-Difluorobenzyl)-1H-1,2,3-triazole-4,5-di(N-acetyl)carboxamide according to claim 1.

**18.** 1-(2,3-Difluorobenzyl)-1H-1,2,3-triazole-4-carbox-amide according to claim 1.

**19.** 1-(2,3-Difluorobenzyl)-1H-1,2,3-triazole-4,5-dicarboxamide according to claim 1.

**20.** 1-(2,4-Difluorobenzyl)-1H-1,2,3-triazole-4,5-dicarboxamide according to claim 1.

**21.** A compound according to any one of claims 1, 2, 5 and 15 to 20 for use in a therapeutic method of treating the human or animal body, for example as an anticonvulsive agent.

**22.** A compound according to any one of claims 3, 4 and 6 to 14 for use in a therapeutic method of treating the human or animal body, for example as an anticonvulsive agent.

**23.** Pharmaceutical compositions containing a compound according to any one of claims 1, 2, 5 and 15 to 21, together with conventional pharmaceutical excipients and/or carriers.

**24.** Pharmaceutical compositions containing a compound according to any one of claims 3, 4, 6 to 14 and 22, together with conventional pharmaceutical excipients and/or carriers.

**25.** A process for the preparation of compounds of formula I according to claim 1, which comprises a) reacting a compound of formula

$$Ph - alk - N_3 \qquad (II)$$

with a compound of formula

$$R_1 - \underset{Y_1}{C} = \underset{Y_2}{C} - R_2 \qquad (III),$$

wherein $Y_1$ is hydroxy and $Y_2$ is hydrogen, or $Y_1$ and $Y_2$ together form an additional bond, or with a salt and/or tautomer thereof, or b) reacting a compound of formula

$$Ph - alk - Z \qquad (IV),$$

wherein Z is reactive esterified hydroxy, with a 1H-1,2,3-triazole derivative of formula

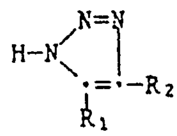

(V)

or with a salt thereof, or c) for the preparation of compounds of formula I wherein either $R_1$ is hydrogen, carbamoyl, N-($C_2$-$C_5$)alkanoylcarbamoyl or N,N-di($C_1$-$C_4$)alkylcarbamoyl and $R_2$ is carbamoyl or N,N-di($C_1$-$C_4$)alkylcarbamoyl, or $R_1$ is carbamoyl or N,N-di($C_1$-$C_4$)alkylcarbamoyl and $R_2$ is carbamoyl, N-($C_2$-$C_5$)alkanoylcarbamoyl or N,N-di($C_1$-$C_4$)alkylcarbamoyl, in a compound of formula

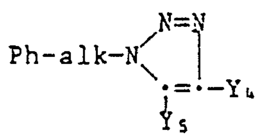

(VI),

wherein $Y_4$ is a radical $Y_A$ which is convertible into carbamoyl or N,N-di($C_1$-$C_4$)alkylcarbamoyl and $Y_5$ is a group $R_1$ or a radical $Y_B$ which is convertible into carbamoyl or N,N-di($C_1$-$C_4$)alkylcarbamoyl, or $Y_4$ is a group $R_2$ and $Y_5$ is a radical $Y_B$ which is convertible into carbamoyl or N,N-di($C_1$-$C_4$)alkylcarbamoyl, converting $Y_A$ and/or $Y_B$ into carbamoyl or N,N-di($C_1$-$C_4$)alkylcarbamoyl, if necessary separating a resulting mixture of isomers into the individual isomers and isolating the isomer of formula I and, if desired, converting a compound of formula I obtained by the process of the invention into another compound of formula I and/or resolving a mixture of enantiomers or diastereoisomers obtained by the process of the invention into the individual components.

**26.** The use of a compound according to any one of claims 1 to 22 for the preparation of an anticonvulsive medicament.

Claims for the following Contracting State:

**1.** A process for the preparation of fluorinated 1-(α-phenylalkyl)-1H-1,2,3-triazole compounds of the formula

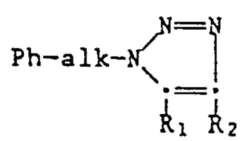

(I),

wherein Ph is an o-fluorinated phenyl radical which may be additionally substituted by up to 2 chlorine atoms inclusive, by 1 fluorine atom and 1 chlorine atom, or by up to 2 fluorine atoms inclusive, alk is methylene, $R_1$ is hydrogen, carbamoyl, N-($C_2$-$C_5$)alkanoylcarbamoyl or N,N-di($C_1$-$C_4$)alkylcarbamoyl, and $R_2$ is carbamoyl, N-($C_2$-$C_5$)alkanoylcarbamoyl or N,N-di($C_1$-$C_4$)alkylcarbamoyl, which process comprises a) reacting a compound of formula

$$Ph - alk - N_3 \qquad (II)$$

with a compound of formula

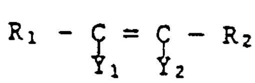

(III),

wherein $y_1$ is hydroxy and $Y_2$ is hydrogen, or $Y_1$ and $Y_2$ together form an additional bond, or with a salt and/or tautomer thereof, or b) reacting a compound of formula

Ph - alk - Z (IV),

wherein Z is reactive esterified hydroxy, with a 1H-1,2,3-triazole derivative of formula

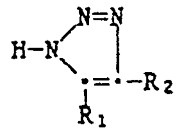

(V)

or with a salt thereof, or c) for the preparation of compounds of formula I wherein either $R_1$ is hydrogen, carbamoyl, N-($C_2$-$C_5$)alkanoylcarbamoyl or N,N-di($C_1$-$C_4$)alkylcarbamoyl and $R_2$ is carbamoyl or N,N-di($C_1$-$C_4$)alkylcarbamoyl, or $R_1$ is carbamoyl or N,N-di($C_1$-$C_4$)alkylcarbamoyl and $R_2$ is carbamoyl, N-($C_2$-$C_5$)alkanoylcarbamoyl or N,N-di-($C_1$-$C_4$)alkylcarbamoyl, in a compound of formula

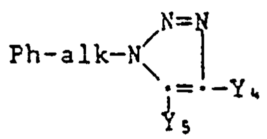

(VI),

wherein $Y_4$ is a radical $Y_A$ which is convertible into carbamoyl or N,N-di($C_1$-$C_4$)alkylcarbamoyl and $Y_5$ is a group $R_1$ or a radical $Y_B$ which is convertible into carbamoyl or N,N-di($C_1$-$C_4$)alkylcarbamoyl, or $Y_4$ is a group $R_2$ and $Y_5$ is a radical $Y_B$ which is convertible into carbamoyl or N,N-di($C_1$-$C_4$)alkylcarbamoyl, converting $Y_A$ and/or $Y_B$ into carbamoyl or N,N-di($C_1$-$C_4$)alkylcarbamoyl, if necessary separating a resulting mixture of isomers into the individual isomers and isolating the isomer of formula I and, if desired, converting a compound of formula I obtained by the process of the invention into another compound of formula I and/or resolving a mixture of enantiomers or diastereoisomers obtained by the process of the invention into the individual components.

**2.** A process according to claim 1, wherein a compound of formula VI wherein $Y_4$ is esterified carboxy and $Y_5$ is hydrogen or is also esterified carboxy is reacted with an excess of ammonia or of a di($C_1$-$C_4$)-alkylamine.

**3.** A process according to claim 1 or 2, wherein compounds of formula I wherein Ph is an o-fluorinated phenyl radical which may be additionally substituted by 1 chlorine atom, by 1 fluorine atom and 1 chlorine atom, or by up to 2 fluorine atoms inclusive, alk is methylene, $R_1$ is hydrogen or a radical $R_2$, and $R_2$ is carbamoyl or N,N-di($C_1$-$C_4$)alkylcarbamoyl are prepared.

**4.** A process according to claim 1 or 2, wherein compounds of formula I wherein Ph is o-fluorophenyl, 2,3-, 2,4-, 2,5- or 2,6-difluorophenyl or 6-chloro-2-fluorophenyl, alk is methylene, $R_1$ is hydrogen or carbamoyl and $R_2$ is carbamoyl are prepared.

**5.** A process according to claim 1 or 2, wherein compounds of formula I wherein Ph is o-fluorophenyl or 2,6-difluorophenyl, alk is methylene, $R_1$ is hydrogen or carbamoyl and $R_2$ is carbamoyl are prepared.

**6.** A process according to claim 1 or 2, wherein compounds of formula I wherein Ph is o-fluorophenyl, 2,5-difluorophenyl, 2,6-difluorophenyl or 6-chloro-2-fluorophenyl, alk is methylene and $R_1$ and $R_2$ are both carbamoyl are prepared.

**7.** A process according to claim 1 or 2, wherein compounds of formula I wherein Ph is 2,6-difluorophenyl, alk is methylene, $R_1$ is hydrogen or carbamoyl and $R_2$ is carbamoyl are prepared.

**8.** A process according to claim 1 or 2, wherein 1-(o-fluorobenzyl)-1H-1,2,3-triazole-4-carboxamide is prepared.

**9.** A process according to claim 1 or 2, wherein 1-(2,6-difluorobenzyl)-1H-1,2,3-triazole-4-carboxamide is prepared.

**10.** A process according to claim 1 or 2, wherein 1-(o-fluorobenzyl)-1H-1,2,3-triazole-4,5-dicarboxamide is prepared.

**11.** A process according to claim 1 or 2, wherein 1-(2,6-difluorobenzyl)-1H-1,2,3-triazole-4,5-dicarboxamide is prepared.

**12.** A process according to claim 1 or 2, wherein 1-(6-chloro-2-fluorobenzyl)-1H-1,2,3-triazole-4-carboxamide is prepared.

**13.** A process according to claim 1 or 2, wherein 1-(6-chloro-2-fluorobenzyl)-1H-1,2,3-triazole-4,5-dicarboxamide is prepared.

**14.** A process according to claim 1 or 2, wherein 1-(2,5-difluorobenzyl)-1H-1,2,3-triazole-4,5-dicarboxamide is prepared.

**15.** A process according to claim 1 or 2, wherein 1-(2,6-difluorobenzyl)-1H-1,2,3-triazole-4,5-di(N,N-dimethyl)-carboxamide is prepared.

**16.** A process according to claim 1 or 2, wherein 1-(2,6-difluorobenzyl)-1H-1,2,3-triazole-4-(N-acetyl)-carboxamide is prepared.

**17.** A process according to claim 1 or 2, wherein 1-(2,6-difluorobenzyl)-1H-1,2,3-triazole-4,5-di(N-acetyl) carboxamide is prepared.

**18.** A process according to claim 1 or 2, wherein 1-(2,3-difluorobenzyl)-1H-1,2,3-triazole-4-carboxamide is prepared.

**19.** A process according to claim 1 or 2, wherein 1-(2,3-difluorobenzyl)-1H-1,2,3-triazole-4,5-dicarboxamide is prepared.

**20.** A process according to claim 1 or 2, wherein 1-(2,4-difluorobenzyl)-1H-1,2,3-triazole-4,5-dicarboxamide is prepared.

**21.** A process for the preparation of pharmaceutical compositions, which comprises mixing a compound of formula I, obtainable according to any one of claims 1 to 20, with conventional pharmaceutical excipients and/or carriers.

## Revendications

**1.** Nouveaux composés fluorés 1-(α -phénylalkyl)1H-1,2,3-triazole de formule

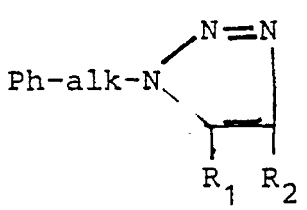

(I),

dans laquelle le Ph en position o représente un phényle substitué par le fluor et substitué le cas échéant en plus par un, jusqu'à 2 atomes de chlore, un atome de fluor et un atome de chlore, ou

jusqu'à 2 atomes de fluor, alk représente le méthylène, $R_1$ l'hydrogène, un carbamyle, N-(alcanoyl $C_2$-$C_5$)carbamyle ou un N,N-di(alkyl $C_1$-$C_4$)carbamyle et $R_2$ un carbamyle, N-(alcanoyl $C_2$-$C_5$)carbamyle, ou un N,N-di(alkyl $C_1$-$C_4$)carbamyle.

2. Composés selon la revendication 1 dans lesquels Ph représente un phényle substitué en position o par un fluor et le cas échéant en plus par 1 atome de chlore, 1 atome de chlore et 1 atome de fluor ou jusqu'à 2 atomes de fluor, alk représente le méthylène, $R_1$ l'hydrogène ou un reste $R_2$ et $R_2$ le carbamyle ou un N,N-di(alkyl $C_1$-$C_4$)carbamyle.

3. Composés selon la revendication 1, dans lesquels Ph représente l'o-fluorophényle, 2,3-, 2,4-, 2,5- ou 2,6-difluorophényle ou le 6-chloro-2-fluorophényle, alk représente le méthylène, $R_1$ l'hydrogène ou le carbamyle et $R_2$ le carbamyle.

4. Composés selon la revendication 1, dans lesquels Ph représente l'o-fluorophényle ou le 2,6-difluorophényle, alk représente le méthylène, $R_1$ l'hydrogène ou le carbamyle et $R_2$ le carbamyle.

5. Composés selon la revendication 1, dans lesquels Ph représente le 2,6-difluorophényle, alk représente le méthylène, $R_1$ l'hydrogène ou un reste $R_2$ et $R_2$ est le carbamyle, un N-(alcanoyl $C_2$-$C_5$)carbamyle ou un N,N-di(alkyl $C_1$-$C_4$)carbamyle.

6. Composés selon la revendication 1, dans lesquels Ph représente l'o-fluorophényle, le 2,5-difluorophényle, le 2,6-difluorophényle ou le 2-chloro-6-fluorophényle, alk représente le méthylène et $R_1$ et $R_2$ représentent tous deux le carbamyle.

7. Composés selon la revendication 1, dans lesquels Ph représente le 2,6-difluorophényle, alk le méthylene, $R_1$ l'hydrogène ou le carbamyle et $R_2$ le carbamyle.

8. 1-(o-fluorobenzyl)-1H-1,2,3-triazol-4-carboxamide selon la revendication 1.

9. 1-(2,6-difluorobenzyl)-1H-1,2,3-triazol-4-carboxamide selon la revendication 1.

10. 1-(o-fluorobenzyl)-1H-1,2,3-triazol-4,5-dicarboxamide selon la revendication 1.

11. 1-(2,6-difluorobenzyl)-1H-1,2,3-triazol-4,5-dicarboxamide selon la rendication 1.

12. 1-(6-chloro-2-fluoro-benzyl)-1H-1,2,3-triazol-4-carboxamide selon la revendication 1.

13. 1-(6-chloro-2-fluoro-benzyl)-1H-1,2,3-triazol-4,5-dicarboxamide selon la revendication 1.

14. 1-(2,5-difluorobenzyl)-1H-1,2,3-triazol-4,5-dicarboxamide selon la revendication 1.

15. 1-(2,6-difluorobenzyl)-1H-1,2,3-triazol-4,5-di(N,N-diméthyl)-carboxamide selon la revendication 1.

16. 1-(2,6-difluorobenzyl)-1H-1,2,3-triazol-4-(N-acétyl)carboxamide selon la revendication 1.

17. 1-(2,6-difluorobenzyl)-1H-1,2,3-triazol-4,5-di(N-acétyl)carboxamide selon la revendication 1.

18. 1-(2,3-difluorobenzyl)-1H-1,2,3-triazol-4-carboxamide selon la revendication 1.

19. 1-(2,3-difluorobenzyl)-1H-1,2,3-triazol-4,5-dicarboxamide selon la revendication 1.

20. 1-(2,4-difluorobenzyl)-1H-1,2,3-triazol-4,5-dicarboxamide selon la revendication 1.

21. Composé selon l'une des revendications 1, 2, 5 et 15 à 20 pour l'utilisation dans un procédé de traitement thérapeutique du corps animal et humain par exemple comme agent anticonvulsion.

22. Composé selon l'une des revendications 3,4 et 6 à 14 pour l'utilisation dans un procédé de traitement thérapeutique du corps animal ou humain, par exemple comme agent anticonvulsion.

**23.** Préparations pharmaceutiques, comprenant un composé selon l'une des revendications 1, 2, 5 et 15 à 21 en plus des véhicules et additifs pharmaceutiques habituels.

**24.** Préparations pharmaceutiques comprenant un composé selon l'une des revendications 3, 4, 6 à 14 et 22 en plus des véhicules et additifs pharmaceutiques habituels.

**25.** Procédé de préparation de composés de formule I selon la revendication 1, caractérisé en ce qu' a) on fait réagir un composé de formule

$$Ph - alk - N_3 \qquad (II)$$

avec un composé de formule

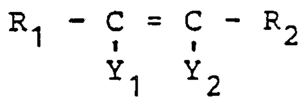

(III)

dans laquelle $Y_1$ est l'hydroxy et $Y_2$ l'hydrogène ou $Y_1$ et $Y_2$ représentent ensemble une liaison supplémentaire, ou un de ses sels et/ou tautomères ou b) un composé de formule

$$Ph - alk - Z \qquad (IV)$$

dans laquelle Z est un hydroxy estérifié pouvant réagir, avec un dérivé 1H-1,2,3-triazole de formule

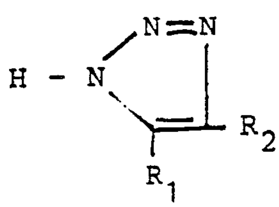

(V)

ou un de ses sels ou c) pour préparer des composés de formule I, dans laquelle soit $R_1$ est l'hydrogène, un carbamyle, un N-(alcanoyl $C_2$-$C_5$)carbamyle ou N,N-di(alkyl $C_1$-$C_4$)carbamyle et $R_2$ un carbamyle ou un N,N-di(alkyl $C_1$-$C_4$)carbamyle, soit $R_1$ est un carbamyle ou un N,N-di(alkyl $C_1$-$C_4$) carbamyle et $R_2$ est un carbamyle, N-(alcanoyl $C_2$-$C_5$) carbamyle ou un N,N-di(alkyl $C_1$-$C_4$), dans un composé de formule

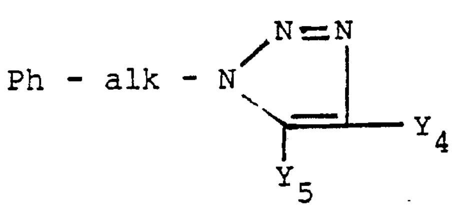

(VI)

dans laquelle $Y_4$ est un reste $Y_A$ transformable en un carbamyle ou N,N-di(alkyl $C_1$-$C_4$)carbamyle et $Y_5$ un groupe $R_1$ ou un reste $Y_B$ transformable en un carbamyle ou ,N-di(alkyl $C_1$-$C_4$)carbamyle, ou $Y_4$ est un groupe $R_2$ et $Y_5$ un reste $Y_B$ transformable en un carbamyle ou N,N-di(alkyl $C_1$-$C_4$) carbamyle, on transforme $Y_A$ et/ou $Y_B$ en un carbamyle ou N,N-di(alkyl $C_1$-$C_4$)carbamyle, si c'est nécessaire on fractionne un mélange d'isomères obtenu en ses composants et on isole l'isomère de formule (I) et si c'est souhaité on transforme un composé de formule I obtenu selon le procédé en un autre composé de formule (I) et/ou on fractionne un mélange d'énantiomères ou bien de diastéréoisomères obtenus selon le procédé, en ses composants.

**26.** Utilisation d'un composé selon l'une ces revendications 1-22 pour préparer un médicament anticonvulsion.

Revendications pour l'Etat contractant suivant : AT

1. Procédé de préparation de composés fluorés 1-(α-phénylalkyl)-1H-1,2,3-triazole de formule

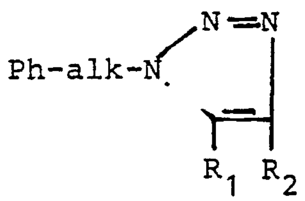 (I),

dans laquelle le Ph en position o représente un phényle substitué par le fluor et substitué le cas échéant en plus par un, jusqu'à 2 atomes de chlore, un atome de fluor et un atome de chlore, ou jusqu'à 2 atomes de fluor, alk représente le méthylène, $R_1$ l'hydrogène, un carbamyle, N-(alcanoyl $C_2$-$C_5$)carbamyle ou un N,N-di(alkyl $C_1$-$C_4$)carbamyle et $R_2$ un carbamyle, N-(alcanoyl $C_2$-$C_5$)carbamyle, ou un N,N-di(alkyl $C_1$-$C_4$)carbamyle,
caractérisé en ce qu'
a) on fait réagir un composé de formule

$$Ph - alk - N_3 \qquad (II)$$

avec un composé de formule

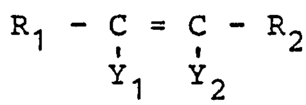 (III)

dans laquelle $Y_1$ est l'hydroxy et $Y_2$ l'hydrogène ou $Y_1$ et $Y_2$ représentent ensemble une liaison supplémentaire, ou un de ses sels et/ou tautomères ou
b) un composé de formule

$$Ph - alk - Z \qquad (IV)$$

dans laquelle Z est un hydroxy estérifié pouvant réagir, avec un dérivé 1H-1,2,3-triazole de formule

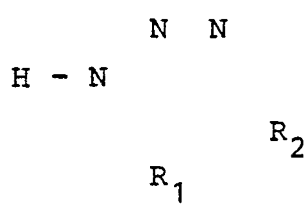 (V)

ou un de ses sels ou c) pour préparer des composés de formule I dans laquelle soit $R_1$ est l'hydrogène, un carbamyle, un N-(alcanoyl $C_2$-$C_5$)carbamyle ou N,N-di(alkyl $C_1$-$C_4$)carbamyle et $R_2$ un carbamyle ou un N,N-di(alkyl $C_1$-$C_4$)carbamyle, soit $R_1$ est un carbamyle ou un N,N-di(alkyl $C_1$-$C_4$)-carbamyle et $R_2$ est un carbamyle, N-(alcanoyl $C_2$-$C_5$)carbamyle ou un N,N-di(alkyl $C_1$-$C_4$), dans un composé de formule

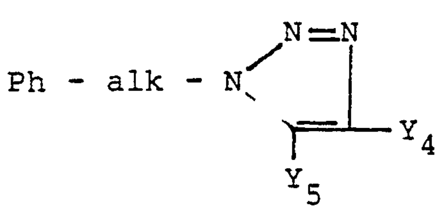 (VI)

dans laquelle $Y_4$ est un reste $Y_A$ transformable en un carbamyle ou N,N-di(alkyl $C_1$-$C_4$)carbamyle et $Y_5$

un groupe $R_1$ ou un reste $Y_B$ transformable en un carbamyle ou N,Ndi(alkyl $C_1$-$C_4$)carbamyle, ou $Y_4$ est un groupe $R_2$ et $Y_5$ un reste $Y_B$ transformable en un carbamyle ou N,N-di(alkyl $C_1$-$C_4$)carbamyle, on transforme $Y_A$ et/ou $Y_B$ en un carbamyle ou N,N-di(alkyl $C_1$-$C_4$)carbamyle, si c'est nécessaire on fractionne un mélange d'isomères obtenu en ses composants et on isole l'isomère de formule (I) et si c'est souhaité on transforme un composé de formule I obtenu selon le procédé en un autre composé de formule (I) et/ou on fractionne un mélange d'énantiomères ou bien de diastéréoisomères obtenus selon le procédé, en ses composants.

**2.** Procédé selon la revendication 1, caractérisé en ce qu'on fait réagir un composé de formule VI dans laquelle $Y_4$ représente un carboxy estérifié et $Y_5$ l'hydrogène, ou également un carboxy estérifié, avec un excès d'ammoniac.

**3.** Procédé selon la revendication 1 ou 2, caractérisé en ce qu'on prépare des composés de formule (I) dans lesquels Ph représente un phényle substitué en position o par un fluor et le cas échéant en plus par 1 atome de chlore, 1 atome de chlore et 1 atome de fluor ou jusqu'à 2 atomes de fluor, alk représente le méthylène, $R_1$ l'hydrogène ou un reste $R_2$ et $R_2$ le carbamyle ou un N,N-di(alkyl $C_1$-$C_4$)-carbamyle.

**4.** Procédé selon la revendication 1 ou 2 caractérisé en ce qu'on prépare des composés de formule (I) dans lesquels Ph représente l'o-fluorophényle, 2,3-, 2,4-, 2,5- ou 2,6-difluorophényle ou le 6-chloro-2-fluoro-phényle, alk représente le méthylène, $R_1$ l'hydrogène ou le carbamyle et $R_2$ le carbamyle.

**5.** Procédé selon la revendication 1 ou 2, caractérisé en ce qu'on prépare des composés de formule (I) dans lesquels Ph représente l'o-fluorophényle ou le 2,6-difluorophényle, alk représente le méthylène, $R_1$ l'hydrogène ou le carbamyle et $R_2$ le carbamyle.

**6.** Procédé selon la revendication 1 ou 2, caractérisé en ce qu'on prépare les composés de formule I, dans lesquels Ph représente l'o-fluorophényle, le 2,5-difluoro-phényle, le 2,6-difluorophényle ou le 6-chloro-2-fluoro-phényle, alk le méthylène et $R_1$ et $R_2$ représentent tous deux le carbamyle.

**7.** Procédé selon la revendication 1 ou 2, caractérisé en ce qu'on prépare les composés de formule I, dans lesquels Ph représente le 2,6-difluorophényle, alk le méthylène, $R_1$ l'hydrogène ou le carbamyle, et $R_2$ le carbamyle.

**8.** Procédé selon la revendication 1 ou 2, caractérisé en ce qu'on prépare le 1-(o-fluorobenzyl)-1H-1-2,3-triazol-4-carboxamide.

**9.** Procédé selon la revendication 1 ou 2, caractérisé en ce qu'on prépare le 1-(2,6-difluorobenzyl)-1H-1,2,3-triazol-4-carboxamide.

**10.** Procédé selon la revendication 1 ou 2, caractérisé en ce qu'on prépare le 1-(o-fluorobenzyl)-1H-1-2,3-triazol-4,5-dicarboxamide.

**11.** Procédé selon la revendication 1 ou 2, caractérisé en ce qu'on prépare le 1-(2,6-difluorobenzyl)-1H-1 ,2,3-triazol-4,5-dicarboxamide.

**12.** Procédé selon la revendication 1 ou 2, caractérisé en ce qu'on prépare le 1-(6-chloro-2-fluoro-benzyl)-1H-1,2,3-triazol-4-carboxamide.

**13.** Procédé selon la revendication 1 ou 2, caractérisé en ce qu'on prépare le 1-(6-chloro-2-fluoro-benzyl)-1H-1,2,3-triazol-4,5-dicarboxamide.

**14.** Procédé selon la revendication 1 cu 2, caractérisé en ce qu'on prépare le 1-(2,5-difluorobenzyl)-1H-1,2,3-triazol-4,5-dicarboxamide.

**15.** Procédé selon la revendication 1 ou 2, caractérisé en ce qu'on prépare le 1-(2,6-difluorobenzyl)-1H-1,2,3-triazol-4,5-di(N,N-diméthyl)-carboxamide.

16. Procédé selon la revendication 1 ou 2, caractérisé en ce qu'on prépare le 1-(2,6-difluorobenzyl)-1H-1 ,2,3-triazol-4-(N-acécyl)carboxamide.

17. Procédé selon la revendication 1 ou 2, caractérisé en ce qu'on prépare le 1-(2,6-difluorobenzyl)-1H-1,2,3-triazol-4,5-di(N-acétyl)carboxamide.

18. Procédé selon la revendication 2, caractérisé en ce qu'on prépare le 1-(2,3-difluorobenzyl)-1H-1,2,3-triazol-4-carboxamide.

19. Procédé selon la revendication 1 ou 2, caractérisé en ce qu'on prépare le 1-(2,3-difluorobenzyl)-1H-1,2,3-triazol-4,5-dicarboxamide.

20. Procédé selon la revendication 1 ou 2, caractérisé en ce qu'on prépare le 1-(2,4-difluorobenzyl)-1H-1,2,3-triazol-4,5-dicarboxamide.

21. Procédé de production de préparations pharmaceutiques, caractérisé en ce qu'on mélange un composé de formule I obtenu selon l'une des revendications 1-20, avec les véhicules et additifs pharmaceutiques usuels.